# EUROPEAN PATENT APPLICATION

(11) **EP 2 034 008 A1**
(43) Date of publication of application: **11.03.2009**
(21) Application number: 07788585.3
(22) Date of filing: 07.06.2007
(51) Int. Cl.: C12N 1/12, C12P 7/64, C12P 7/20, C12P 5/00, C12P 1/00, C10L 1/00, B01D 53/84

(54) **METHOD FOR OBTAINING ENERGY-GENERATING COMPOUNDS BY MEANS OF ELECTROMAGNETIC ENERGY**

(30) Priority: 09.06.2006 ES 200601568
(71) Applicant: Stroïazzo-Mougin, Bernard A. J., 03560 El Campello (Alicante) (ES); Gomis Catala, Cristian, 03560 El Campello Alicante (ES)
(72) Inventor: Stroïazzo-Mougin, Bernard A. J., 03560 El Campello (Alicante) (ES); Gomis Catala, Cristian, 03560 El Campello Alicante (ES)
(74) Representative: Elzaburu Marquez, Alberto
(86) International application number: PCT/ES2007/000337
(87) International publication number: WO 2007/144441

(57) **Abstract**

The present invention relates to a process for obtaining energetic compounds by means of electromagnetic energy produced by sunlight or artificial light by means of using phytoplankton cultures.

## Description

### Technical Field of the Invention

The present invention relates to a process for obtaining energetic compounds by means of electromagnetic energy produced by sunlight or artificial light by means of the use of phytoplankton cultures.

The invention is comprised within the technical field of the exploitation of renewable energy by means of the action of phytoplankton organisms that normally belong to the following taxonomic families: Chlorophyceae, Bacillariophyceae, Dinophyceae, Cryptophyceae, Chrysophyceae, Haptophyceae, Prasinophyceae, Raphidophyceae ... generally the taxonomic families comprising species of the chromophyte division, all of them characterized by being flagellated or nonflagellated single-celled organisms and with a strictly planktonic (holoplanktonic) life phase, or at least one of its phases being planktonic (meroplanktonic).

Biofuels, byproducts such as naphthas, kerosene, thermal energy, electric energy...are particularly obtained by means of the present process.

### State of the Art

Up until now, biofuels have been obtained from higher plant cultures, usually from the group of phanerogams or flowering plants (sunflowers, palm, European palm,..), and usually on the terrestrial surface (land plants).

The obligation for the economic zones to comply with the objectives imposed by the Kyoto protocol on the reduction of CO₂/SO₂ emissions and the emission of other gases causing the so-called greenhouse effect is forcing countries to search for alternative and renewable fuels to prevent possible penal taxes.

Although the production of solar and wind energy is increasing in some regions, these technologies are very expensive and are not viable in all climatic areas. In these conditions, biofuels have an important role as substitutes of fossil fuels, especially in transport and heating applications.

The production costs of biofuels from plants, such as palm and rapeseed oil have always been a reason for concern. Taking into account the low oil production indexes per hectare, enormous amounts of resources would be needed to reach commercial production. Land and water are two limited resources and it is preferable to use them to produce food products, which are furthermore more profitable for farmers. Intensive fertilization is furthermore a form of land and water pollution of the first order. Extensive single crop farming is also one of the main enemies of biodiversity.

Phytoplankton represents a viable solution to the aforementioned problem given that about 50% of the dry mass of single-celled organisms can generally be transformed into biofuel. In addition, the annual production per hectare of biofuel is 40 times higher than with the next most cost-effective product, palm oil. A drawback is that the production of phytoplankton oil requires covering large areas of land with rather shallow water, as well as introducing large amounts of CO₂, an essential element for phytoplankton to produce oil. Open algological production systems have a relatively low cost, but the harvesting process is very laborious and therefore expensive. On the other hand, phytoplankton culturing is carried out in open systems, which makes it vulnerable to pollution and to problems for cultures, which can lead to total production loss. In this same sense, an advantage of the process described in the present invention is that the system is kept closed and in conditions such that the culture is not contaminated by bacteria, fungi...because in addition to being closed, the culture is enriched by means of nutrients, and fungicides and antibiotics are incorporated so that it is axenic.

Many patents show processes for obtaining biofuels by means of plants as is the case of patent ES2192978 "Process for elaborating biofuel for diesel engines from methyl esters of *Brassica carinata* oil without erucic acid" or patent ES2201894 "Process for producing biodiesel fuels with improved properties at low temperature", or patent ES2207415 "Versatile biofuel usable in any type of conventional internal combustion engine or burner" or patent ES2245270 "Process for obtaining fuel for diesel cycle engines from used vegetable oils". However, none of these patents describes a process as novel as that described in the present invention because, compared to the rest of the systems described in the state of the art, this invention is carried out in closed systems and therefore with absolute control of the reaction conditions. It is not carried out by means of conventional systems using plants and vegetables such as soybean, rapeseed, palm oil...,although it is true that biofuels can be obtained from such systems, the biofuels are generated in an infinitely smaller amount than by means of phytoplankton, which is one of the objects of the present invention.

Patent application WO 03/094598 A1 entitled "Photobioreactor and process for biomass production and mitigation of pollutants in flue gases" describes a generic photobioreactor mainly focused on decontaminating COx, SOx and NOx type gases. It is basically a system working in a discontinuous manner (distinguishing between day/night photoperiods) and is open, its liquid medium not being axenic. It does not control nitrogen and carbon dioxide concentrations for the purpose of increasing biofuel production. It is not designed to work with monospecific or monoclonal algae strains. Its design does not contemplate biofuel production as the main objective, rather it is focused on gas purification. On the other hand, as regards the photosynthetic compounds it refers to, it does not demand conditions disabling the system.

Compared to the present invention object of the patent, a completely novel system is set forth, based in contrast on the following features:
- It is completely closed.
- It is completely axenic.
- It works continuously.
- It works with monospecific and monoclonal strains.
- It does not accept any photosynthetic organism, but rather it at least requires that they are not organisms forming biofouling on the inner surface of the photoconverter.
- It requires that the phytoplankton species do not form colonies.
- It requires that the phytoplankton species do not generate exo-mucilage.
- It requires that the cultured species contains at least 5% of fatty acids.
- It enhances the use of nonflagellated and floating phytoplankton species.
- It does not accept any type of liquids as culture medium, it focuses on freshwater, brackish water and sea water.
- Its main objective is to obtain metabolic synthesis compounds with energetic properties or with pre-energetic properties essentially aimed at obtaining biofuels.

On the other hand, up until now there has been no process of this type whereby in addition to biodiesel, free from any type of contamination, other, though no less important, byproducts can be obtained, such as kerosenes, naphthas, fertilizers, glycerin, electric energy, part of which will be useful for supplying the necessary energy to the system, heat energy which will be useful as a source for developing seawater desalination processes...

Another advantage of the process described in the present invention is that it is carried out continuously, such that it ensures 24-hour a day biomass productivity by phytoplankton compared to the systems described in the literature which work in discontinuous conditions, and therefore system efficiency and productivity is determined by sunlight hours and by the geographical location in which the present process if carried out.

This entire process can be carried out by means of the action of phytoplankton which carries out photosynthesis by means of capturing solar energy, carbon dioxide and water. The enrichment by nutrients and an extraordinary supply of CO₂ (usually obtained from the pyrolysis of urban solid waste, industrial solid waste and plant origin biomass) will contribute to the increase of plankton production and therefore of the concentration of precursor compounds for obtaining biofuels. Therefore, the whole group is a process for the exploitation of clean energies such as solar energy, and as a result, obtaining a recycling of the waste materials which will be the essential carbon and nitrogen source for the phytoplankton to produce carbohydrates, lipids and other secondary compounds. Pure glycerin and biodiesel, among other compounds, are obtained thanks to this process and by carrying out transesterification reactions on the lipids and a subsequent separation.

### Description

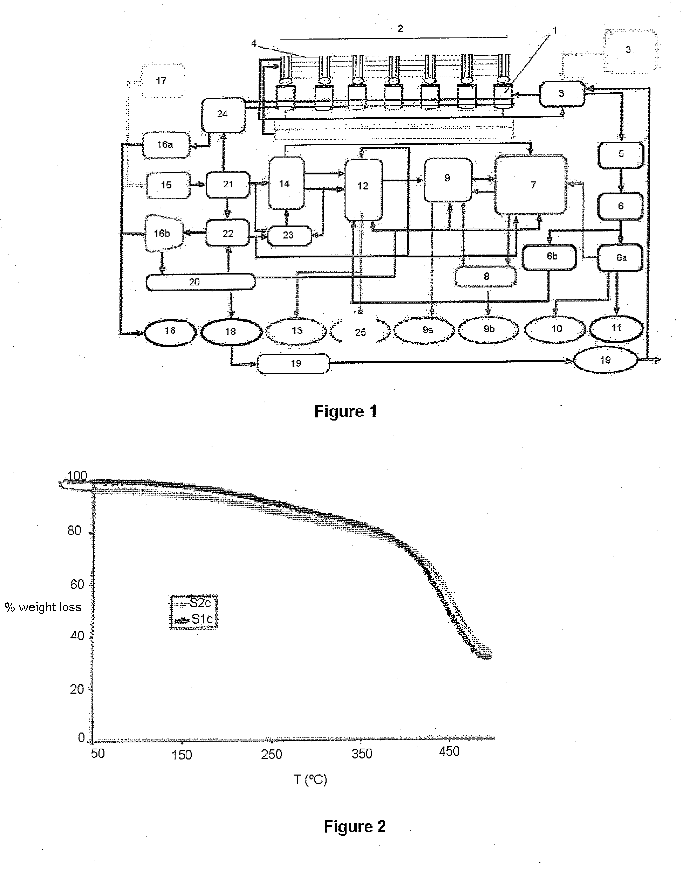

A main first aspect of the present invention is a process for obtaining energetic compounds by means of electromagnetic energy as shown in Figure 1, characterized in that said process is carried out in a mass production photoconverter (1) working continuously and which is a closed system, i.e. a system working 24 hours a day.

In the photoconverter (1) for obtaining energetic compounds by means of electromagnetic energy there is a controlled phytoplankton culture, phytoplankton being a plant species having the peculiarity that they are individual cells having an efficacy and efficiency much greater than that which can be considered with any live multicelled organism. The phytoplankton used in the present process is based on single-celled algae strains which are used to feed rotifers and artemia in the breeding of young fish in fish farms existing in the world. Phytoplankton generally has a fat and carbohydrate concentration ranging between 5 and 80% of its dry weight (according to the genus and species), and its mass culture or growth is useful for obtaining biofuels and reducing atmospheric CO₂, instead of being used for feeding in processes related to breeding fish and other aquatic organisms.

In a non-limiting sense, the phytoplankton community used in the present process usually belongs to the following taxonomic families Chlorophyceae, Bacillariophyceae, Dinophyceae, Cryptophyceae, Chrysophyceae, Haptophyceae, Prasinophyceae, Raphidophyceae ... generally the taxonomic families comprising species of the chromophyte division, all of them characterized by being flagellated or nonflagellated single-celled organisms and with a strictly planktonic (holoplanktonic) life phase, or at least one of its phases being planktonic (meroplanktonic).

The present phytoplankton is cultured by means of adding the necessary nutrients and the culture is axenic and monospecific, an axenic culture understood as a culture free from contamination by bacteria, fungi...

The process carried out in the present invention is characterized in that it has the following steps:

The phytoplankton is cultured in photoconverters (1) such that they are exposed to both natural and artificial electromagnetic radiation (2). In this same sense, the electromagnetic energy supplied comprises spectrum wavelengths ranging from 430 to 690 nm.

In this same sense, for the culture to develop, the necessary nutrients (3) for the growth thereof are injected. The phytoplankton thus carries out photosynthesis (4) and begins producing biomass, which is subsequently separated from the water (5) by means of techniques known in the state of the art, but said separation is mainly carried out by means of centrifugation, flocculation, concentration and/or evaporation.

The phytoplankton culturing conditions in the photoconverters are the following:
- Temperature: from 15 to 35°C, preferably from 20 to 30°C.
- Sunlight intensity: 600 to 800 watts/m².
- Artificial light intensity: 4 to 30 watts/m².
- Salinity: 0 per thousand up to 50 per thousand.
- Phytoplankton concentration in the culture medium: from 1,000,000 cells/ml up to 400,000,000 cells/ml.
- pH: from 7 to 8.4.

The biomass produced by the phytoplankton community after photosynthesis will mainly and essentially contain lipids (fatty acids) (6a), carbohydrates (6b), pigments, silicates, celluloses, hemicelluloses and generally products derived from the secondary metabolism of cultured phytoplankton plants.

The lipids and carbohydrates produced by phytoplankton as part of its biomass are then separated and extracted (6) from the remaining biomass by means of using organic solvents (hexane type solvents or the like) and/or techniques of molecular dispersion, critical point, vapor pressure,...

Two paths can be followed with the lipids obtained in the previous step, one of such paths is to carry out transesterification (7), such that glycerin, biofuel and byproducts, which are no less important than the previous products, are obtained.

In addition, the lipids extracted and separated from the biomass produced by the phytoplankton can be reused to obtain industrial fuel (10) and fertilizers (11) from them.

Transesterification is understood as a chemical process whereby the alkoxy group of an ester is exchanged for another alcohol, as shown in the following reaction:

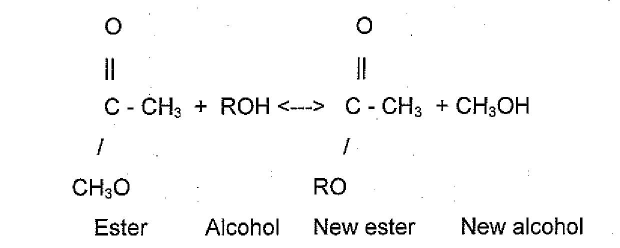

To carry out the transesterification of the lipids obtained as part of the biomass produced by the phytoplankton described in the present process, ethanol and methanol from a Fischer Tropsch-type reactor (14) are used, which reactor is in turn fed by means of synthesis gas (23), steam and heat from a pyrolysis gasifier (15) for biomass (17) or waste from dumps. On the other hand, the pyrolysis gasifier for biomass and the waste from dumps produce CO₂ and NOₓ, which are part of the nutrients used for feeding and growing cultured phytoplankton by means of the present described process, are used in (1) after their passage through a gas cleaner (24). The excess gases of the pyrolysis gasifier are furthermore reused to obtain electric energy (16) through turbines (16a and 16b) and a condenser (20) and thermal energy (18) passing through a heat exchanger (21) and steam boiler (22), which is reused to desalinate (19).

A Fischer Tropsch-type reactor is understood as a reactor in which the synthesis gas is converted into long linear paraffin chains, light olefins, and water. Liquid hydrocarbons are thus obtained, the current of hydrocarbons produced is refined and they are separated into end products. By means of this process, the end products obtained are free from sulfur and aromatic compounds, making them highly desirable. The biofuel obtained further has a high octane number, which makes is compatible with the specifications for clean diesel.

A pyrolysis gasifier is understood as a gasifier which allows using and recycling most of the available waste. The process allows treating segregated or mixed urban and industrial waste. It is also technically possible, without any additional difficulties, to treat toxic waste, hospital waste, tires in the same industrial plants, i.e. to propose an overall definitive solution to the problems set forth by waste. The different advantages include the fact that the process is economically viable and less expensive than any other process, and particularly, incineration, thermolysis, methanation processes, and the fact that the process is completely ecological, without any environmental impact and offers a definitive solution to the waste problem, i.e., a "zero dump" solution. The pyrolysis gasifier achieves that all the waste is transformed into synthesis gas, hydrochloric acid, hydrofluoric acid and hydrogen sulfide, the latter three being three products which can be relatively easily made inert.

A synthesis gas is understood as a gas formed by a mixture of carbon monoxide, hereinafter CO, and hydrogen, hereinafter H₂, which is obtained from a mixture of methane, hereinafter CH₄, and oxygen, hereinafter O₂, obtained from the air and steam by means of a pyrolysis gasifier.

After this point, (8) the biofuel is separated from the glycerin (9b) and the remaining byproducts by means of density gradients, such that 3 fractions are obtained, which are biofuel (9a) obtained through a percolator (9), mainly for the use in the automotive industry, glycerin and the byproducts.

The biofuel concentration obtained according to the process carried out in the present invention is from 0.7 g/l to 8 g/l. Biofuel concentrations from 0.7 g/l to 6 g/l are preferably obtained.

The carbohydrates obtained from the biomass from the phytoplankton are subjected to catalysis (12), such that naphthas (13), kerosenes (25), polymers and gases from the catalysis of carbohydrates are obtained.

According to another essential aspect of the present invention, the nutrients provided to the phytoplankton culture present in the photoconverter are previously ionized such that they are fixed and assimilated more quickly and effectively. The nutrients injected in the present process are carbon dioxide, hereinafter CO₂, NOx (in the form of nitrate, nitrites, ammonium, nitrous oxide,...), vitamins, antibiotics, fungicides, water, trace elements and orthophosphoric acid.

The antibiotics added to the culture are a mixture of penicillin and streptomycin at a concentration range from 100 to 300 mg/l each, preferably at a concentration range from 150 to 250 mg/l and more preferably at a concentration of 200 mg/l for each of the components of the mixture.

The fungicides added to the culture are a mixture of griseofulvin and nystatin at a concentration range from 100 to 300 mg/l each, preferably at a concentration range from 150 to 250 mg/l and more preferably at a concentration of 200 mg/l for each of the components of the mixture.

The water added to the phytoplankton culture can be freshwater, brackish water, or salt water.

A series of embodiments, having no limiting sense in relation to that expressed in the present process object of the invention, are set forth below.

### Brief Description of the Drawings

Figure 1 shows the scheme of the process for obtaining energetic compounds by means of electromagnetic energy by means of photoconverters and the use of phytoplankton.
Figure 2 shows the graphic representation of the thermogravimetries conducted in the embodiment where Slc is the sample S1 which has been subjected to an ultrasound treatment, a separation of the solids in suspension by centrifugation and a treatment of the latter with n-hexane. S2c is the sample S2 which has been subjected to an ultrasound treatment, a separation of the solids in suspension by centrifugation and a treatment of the latter with n-hexane.
Figure 3 shows a graphic representation of the chromatograms obtained for the extracts of the samples centrifuged and treated with distilled water from the embodiment (S1a and S2a), where S1a represents the sample S1 which has been subjected to centrifugation, addition of distilled water and addition of n-hexane. S2a represents the sample S2 which has been subjected to centrifugation, addition of distilled water and addition of n-hexane. The time in minutes is measured on the x-axis and the absorbance is measured on the y-axis.

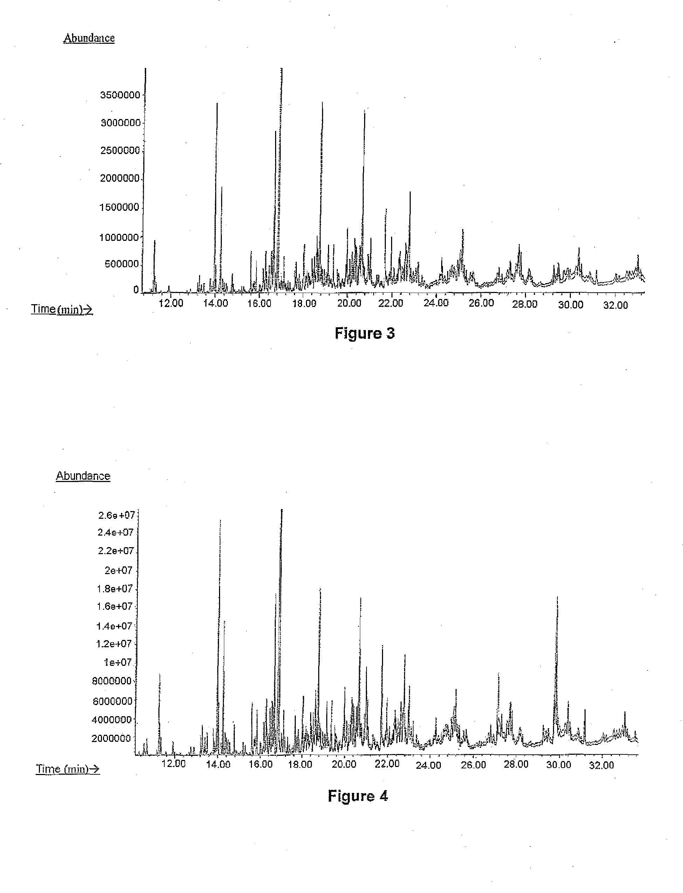

Figure 4 shows the graphic representation of the chromatograms obtained for the extracts of the samples centrifuged and not treated with distilled water from the embodiment (S1c and S2c), where Slc is the sample S1 which has been subjected to an ultrasound treatment, a separation of the solids in suspension by centrifugation and a treatment of the latter with n-hexane. S2c is the sample S2 which has been subjected to an ultrasound treatment, a separation of the solids in suspension by centrifugation and a treatment of the latter with n-hexane. The time in minutes is measured on the x-axis and the absorbance is measured on the y-axis.

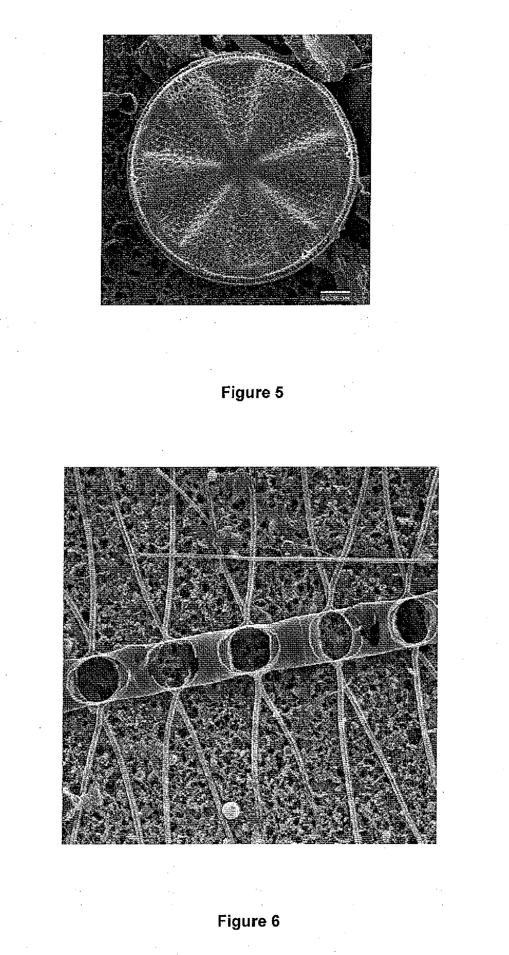

Figure 5 shows the scanning electron microphotographs of *Actinodiscus sp.*
Figure 6 shows the scanning electron microphotographs of *Chaetoceros sp.*

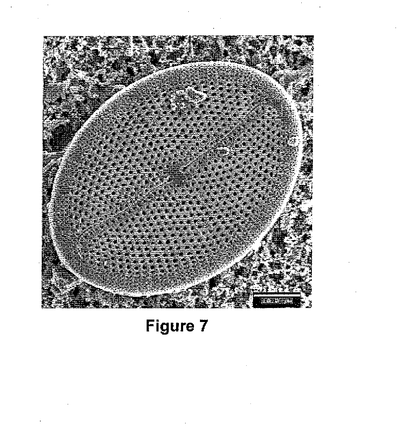

Figure 7 shows the scanning electron microphotographs of *Cocconeis sp.*

### Embodiments

### Obtaining fatty acids from phytoplankton

- Two samples consisting of a phytoplankton suspension in salt water were studied. Said samples are called Sl and S2.
- The possibility of extracting the fatty acids generated by the phytoplankton by means of the extraction in the following conditions was studied:
   - By separating the solids suspended in the samples, treating with distilled water and later adding solvent to the aqueous phase.
   - By separating the solids suspended in the samples and later directly treating them with the solvent.
   - Two solvents were tested: dichloromethane and hexane.
   - Some samples were subjected to ultrasounds for the purpose of studying the influence of this technique on the extraction of soluble substances in the organic phase.
- Two processes were tested for separating the suspended solids from the salt water matrix: filtration and centrifugation. In the conditions used, the separation by filtration was not effective because the solid materials blocked the filters. Good results were obtained by means of separation by centrifugation in the following conditions:
   - rotation speed: 1700 rpm
   - time = 10 minutes
   - temperature = 27-32°C
- The organic phases from all the tests conducted were analyzed by means of gas chromatography, with the detection of the compounds separated in the chromatograph by means of mass spectrometry (GC/MS).
- The following samples were analyzed:
   - S1a sample S1, centrifugation; addition of distilled water; addition of n-hexane.
   - S2a: sample S2, centrifugation; addition of distilled water; addition of n-hexane.
   - Slb: sample S1; centrifugation; addition of distilled water; addition of diclorometano.
   - S2b: sample S2; centrifugation; addition of distilled water; addition of diclorometano.
   - Slc: sample S1; treatment with ultrasounds; separation of the solids in suspension by centrifugation; treatment of the latter with n-hexane.
   - S2c: sample S2; treatment with ultrasounds; separation of the solids in suspension by centrifugation; treatment of the latter with n-hexane.
   - S1 E: sample S1; treatment with ultrasounds; addition of n-hexane.
   - S2V: sample S2; heating; addition of n-hexane.

The extracts from samples S1c and S2c were studied by means of thermogravimetry (pyrolysis from 50°C to 500°C at a heating rate of 20°C/mm in a nitrogen atmosphere). The results were very similar for both. It was verified that the non-volatile fraction contained in these extracts was about 0.001-0.003 g of material/g of culture. At 500°C a non-volatile residue was obtained which meant about 30-35% of the pyrolyzed mass. Figure 2 shows the thermographs obtained.

The following figures show, by way of example, the chromatograms obtained for samples (S1a and S2a) and (S1c and S2c), respectively. As can be seen, the extract obtained when n-hexane was used as a solvent has a great variety of compounds and the spectra are very similar in all the cases. The identification of the peaks showed the existence of a great variety of essentially linear saturated hydrocarbons. The appearance of peaks corresponding to fatty acids (hexadecanoic or palmitic acid at 20,986 mm and octadecanoic or stearic acid at 22,986 mm) as well as glyceryl stearate (29,795 mm) was also observed. On the other hand, the great similarity between the chromatograms obtained for the extracts of the samples centrifuged and treated with distilled water (S1a and S2a) (Figure 3) and not treated with distilled water (S1c and S2c) (Figure 4) shows that it is not necessary to add water to favor the release of soluble substances in n-hexane by the cells, therefore the methodology to be applied in future tests will consists of the direct treatment of the solids in suspension separated after the centrifugation step.

On the other hand, the chromatograms corresponding to the extracts obtained from the samples treated with dichloromethane had a considerably smaller number of peaks than those obtained in the presence of n-hexane, thus indicating the lower extraction capacity of dichloromethane, which is therefore discarded for its use in future tests.

The chromatograms of the extracts corresponding to samples SIE and S2V also showed the appearance of a relatively small number of peaks compared to those obtained for samples S1a, S2a, Slc and S2c, which showed the need to separate phytoplankton from the saline suspension in which it is found as a step prior to the addition of the extraction agent.

• The results obtained in the previous tests suggest the possibility of extracting between 1 and 2 g of organic compounds (soluble in n-hexane) per kg of culture. According to the thermogravimetry tests, about 65-70% of this amount can be thermally degraded under 500°C, whereas the GC/MS analyses show the presence of a great variety of hydrocarbons as well as several fatty acids and their corresponding esters. Therefore, in view of these values, it is to be expected that up to about 0.7 g of potentially usable materials per kg of culture (i.e. per kg of phytoplankton suspension in the salt water) can be obtained, which is very interesting from the energetic point of view.

The following operating conditions have been selected in order to complete this phase of preliminary tests, and without discarding the need for a systematic study to determine the optimal methodology for the separating the compounds generated by the phytoplankton:
1. Separation of the phytoplankton from the suspension in which it is located by means of centrifuging and later decanting the supernatant liquid.
2. Addition of n-hexane to the solid phase from the previous step. In this step, the following must be evaluated: a) the optimal ratio between the respective amounts of hexane and phytoplankton, b) the time and type of contact allowing the best separation (in the previous tests, the mixtures were left in test tubes for 2-4 days and were manually stirred at irregular time intervals), c) the suitability of a drying step or of not carrying out a drying step on the phytoplankton prior to the addition of solvent.
3. Analysis of the phase extracted by different techniques.

## Claims

1. A process for obtaining energetic compounds by means of electromagnetic energy, **characterized in that** comprises the following steps:
a. culturing the phytoplankton in photoconverters (1);
b. exposing the phytoplankton to electromagnetic energy (2);
c. injecting nutrients (3);
d. carrying out photosynthesis by the phytoplankton (4);
e. producing biomass by the phytoplankton;
f. separating water from the phytoplankton (5);
g. separating and/or extracting (6) the lipids (6a) and carbohydrates (6b) from the biomass obtained from the phytoplankton;
h. transesterifying (7) the lipids obtained in step g);
i. separating the glycerin (9b) and byproducts from the biofuel through a separator (8);
j. filtering through a percolator (9) to obtain biofuel (9a);
k. treating the lipids obtained in step g) to obtain industrial fuel (10) and fertilizers (11);
l. catalysis (12) of the carbohydrates obtained in step g);and
m. separating naphthas (13), kerosenes (25), polymers and gases from the catalysis of step.
n. gasifying by pyrolysis (15) the biomass (17) to obtain synthesis gas (23), steam and heat.

2. A process for obtaining energetic compounds by means of electromagnetic energy according to any of the previous claims, **characterized in that** the photoconverter (1) works continuously and closed to the exterior.

3. A process for obtaining energetic compounds by means of electromagnetic energy according to any of the previous claims, **characterized in that** the phytoplankton culture is axenic and monospecific.

4. A process for obtaining energetic compounds by means of electromagnetic energy according to claim 1, **characterized in that** in step f), the separation of water from phytoplankton (5) is carried out by means of centrifugation, flocculation, concentration and/or evaporation.

5. A process for obtaining energetic compounds by means of electromagnetic energy according to claim 1, **characterized in that** in step i), the separation of biofuel (9a) from glycerin (9b) and remaining byproducts is carried out by means of density gradients.

6. A process for obtaining energetic compounds by means of electromagnetic energy according to claim 1, **characterized in that** the biofuel (9a) obtained in step j) is at a concentration of 0.7 g/l to 8 g/l.

7. A process for obtaining energetic compounds by means of electromagnetic energy according to claim 1, **characterized in that** the biofuel (9a) obtained in step j is at a concentration of 0.7 to 6 g/l.

8. A process for obtaining energetic compounds by means of electromagnetic energy according to claim 1, **characterized in that** in step b), the electromagnetic energy (2) comes form sunlight and/or artificial electric light.

9. A process for obtaining energetic compounds by means of electromagnetic energy according to claim 1, **characterized in that** in step c) the nutrients (3) are CO₂, NO_{X}, vitamins, antibiotics, fungicides, water, trace elements and phosphates.

10. A process for obtaining energetic compounds by means of electromagnetic energy according to the previous claim, **characterized in that** the nutrients (3) are ionized.

11. A process for obtaining energetic compounds by means of electromagnetic energy according to claim 9, **characterized in that** the antibiotics are of the group formed by a mixture of penicillin/streptomycin.

12. A process for obtaining energetic compounds by means of electromagnetic energy according to claim 11, **characterized in that** the antibiotics are each at a concentration range from 100 to 300 mg/l.

13. A process for obtaining energetic compounds by means of electromagnetic energy according to claim 11, **characterized in that** the are each at a concentration range from 150 to 250 mg/l.

14. A process for obtaining energetic compounds by means of electromagnetic energy according to claim 11, **characterized in that** the antibiotics are each at a concentration of 200 mg/l.

15. A process for obtaining energetic compounds by means of electromagnetic energy according to claim 9, **characterized in that** the fungicides are of the group formed by a mixture of griseofulvin/nystatin.

16. A process for obtaining energetic compounds by means of electromagnetic energy according to claim 15, **characterized in that** the fungicides are each at a concentration range from 100 to 300 mg/l.

17. A process for obtaining energetic compounds by means of electromagnetic energy according to claim 15, **characterized in that** the fungicides are each at a concentration range from 150 to 250 mg/l.

18. A process for obtaining energetic compounds by means of electromagnetic energy according to claim 15, **characterized in that** the fungicides are each at a concentration of 200 mg/l.

19. A process for obtaining energetic compounds by means of electromagnetic energy according to claim 9, **characterized in that** the water is of the freshwater, brackish water and/or salt water type.

20. A process for obtaining energetic compounds by means of electromagnetic energy according to claim 1, **characterized in that** the electromagnetic energy (2) is at a range of spectrum wavelengths from 430 to 690 nanometers.

21. A process for obtaining energetic compounds by means of electromagnetic energy according to claim 1, **characterized in that** the biomass obtained in step e) is formed by lipids (6a), carbohydrates (6b), pigments, silicates and products derived from the secondary metabolism of the cultured phytoplankton.

22. A process for obtaining energetic compounds by means of electromagnetic energy according to claim 1, **characterized in that** the separation of the lipids (6a) and carbohydrates (6b) of step g) is carried out by means of hexane and/or molecular dispersion.

23. A process for obtaining energetic compounds by means of electromagnetic energy according to claim 1, **characterized in that** in step h) the transesterification (7) is carried out by means of using ethanol and methanol from a Fischer Tropsch-type reactor (14).

24. A process for obtaining energetic compounds by means of electromagnetic energy according to the previous claim, **characterized in that** the Fischer Tropsch-type reactor (14) is fed by means of synthesis gas (23), steam and heat from a pyrolysis gasifier (15) for biomass (17) or waste from dumps.

25. A process for obtaining energetic compounds by means of electromagnetic energy according to claims 1 and 9, **characterized in that** the CO₂ and NO_{X} are used in (1) after their passage through a gas cleaner (24) and come from a pyrolysis gasifier (15) among other products as excess gases.

26. A process for obtaining energetic compounds by means of electromagnetic energy according to the previous claim, **characterized in that** the excess gases of the pyrolysis gasifier (15) are reused to obtain electric energy (16) through turbines (16a and 16b) and a condenser (20).

27. A process for obtaining energetic compounds by means of electromagnetic energy according to claim 25, **characterized in that** the excess gases of the pyrolysis gasifier (15) are reused to obtain thermal energy (18) passing through a heat exchanger (21) and a steam boiler (22).

28. A process for obtaining energetic compounds by means of electromagnetic energy according to the previous claim, **characterized in that** the thermal energy obtained is used to desalinate water (19).
